# EUROPEAN PATENT APPLICATION

(11) **EP 3 895 609 A1**
(43) Date of publication of application: **20.10.2021**
(21) Application number: 19897102.0
(22) Date of filing: 22.11.2019
(51) Int. Cl.: A61B 5/0478

(54) **BIOLOGICAL ELECTRODE AND BIOLOGICAL ELECTRODE PRODUCTION METHOD**

(30) Priority: 10.12.2018 JP 2018231021
(71) Applicant: NOK Corporation, Minato-ku Tokyo 105-8585 (JP)
(72) Inventor: YOSHITOMI Takumi, Tsujido-shinmachi, Fujisawa-shi, Kanagawa 251- 0042 (JP); FUTASHIMA Ryo, Tsujido-shinmachi, Fujisawa-shi, Kanagawa 251- 0042 (JP); UDA Toru, Tokyo 105-0004 (JP)
(74) Representative: TBK
(86) International application number: PCT/JP2019/045733
(87) International publication number: WO 2020/121777

(57) **Abstract**

Provided are a bioelectrode, an electrode portion and a lead wire (connection wire) portion of which can be stably connected, and a manufacturing method of the bioelectrode.

A bioelectrode (1) includes a support member (10), an electrode member (20) of conductive rubber, including a supported part (21) that is a member to be supported by the support member (10) and at least one electrode part (24) that is a member protruding from the supported part (21), and a snap-button-type connector (30) that is provided on the support member (10), and that electrically connects the electrode member (20) to outside. The snap-button-type connector (30) and the supported part (21) are integrally molded.

## Description

### Technical Field

The present invention relates to a bioelectrode and a manufacturing method of the bioelectrode, and particularly relates to a bioelectrode for detecting biosignals such as brain waves, and a manufacturing method of the bioelectrode.

### Background Art

Conventionally, a bioelectrode is used to detect biosignals. The bioelectrode is used in contact with a body of a subject. For example, the bioelectrode is used to detect brain wave signals for analysis of brain function with the aim of early detection of conditions such as Alzheimer's disease.

A bioelectrode for brain wave detection is used to detect brain wave signals, by having an electrode member placed in direct contact with a scalp of a subject. Conventional bioelectrodes include those that are thin plates having a dish shape, the thin plates being of highly conductive metal such as silver or gold. Such a bioelectrode that is a thin plate has poor adhesion to skin, and gel, cream, paste or the like has to be applied between skin and the bioelectrode to reduce contact impedance to the skin. The applied substances have to be removed after detection of biosignals, and use thereof is burdensome.

Furthermore, an electrical double layer is formed at an interface between skin and an electrode due to ionization of metal, and a polarization voltage is generated. Fluctuations in the polarization voltage causes a baseline drift in a signal, and to stabilize the polarization voltage, aging of forming a silver chloride film on an electrode surface has to be performed with respect to a silver electrode.

In contrast, bioelectrodes that do not need application of gel or the like include those that use a probe made of metal (for example, see Patent Literature 1), or those that are formed by impregnating a water absorbing member such as sponge with electrolyte solution in which amino acid or organic salt is dissolved (for example, see Patent Literature 2).

### Document List

### Patent Literatures

Patent Literature 1: Japanese Patent Application Publication No. 2013-248306
Patent Literature 2: Japanese Patent Application Publication No. 2013-144051

### Summary of Invention

### Technical Problem

With a conventional bioelectrode that uses silver electrode/silver chloride electrode, an electrode portion and a lead wire (connection wire) portion are integrated with each other. However, with a bioelectrode that does not need application of gel or the like, the electrode portion and the lead wire portion are separate. Accordingly, a connection portion between the electrode portion and the lead wire portion is unstable, and the connection portion between the electrode portion and the lead wire portion possibly comes apart due to movement of a subject or the like. Accordingly, with respect to a conventional bioelectrode, a structure with which the electrode portion and the lead wire portion can be stably connected is desired.

The present invention has been made in view of the problems described above, and it is an object of the present invention to provide a bioelectrode, an electrode portion and a lead wire (connection wire) portion of which can be stably connected, and a manufacturing method of the bioelectrode.

### Solution to Problem

To achieve the object described above, a bioelectrode according to the present invention is characterized by including: a support member; an electrode member of conductive rubber, including a supported part that is a member to be supported by the support member, and at least one electrode part that is a member protruding from the supported part; and a snap-button-type connector that is provided on the support member, and that electrically connects the electrode member to outside, where the snap-button-type connector and the supported part are integrally molded.

With the bioelectrode according to an aspect of the present invention, the snap-button-type connector includes a supported-part-side fitting part provided on a side of the supported part, and an outer-side fitting part provided on an outer side, the supported-part-side fitting part includes a supported-part-side-fitting-part base portion, and a protruding fitting part that is a member protruding from the supported-part-side-fitting-part base portion, the outer-side fitting part includes an outer-side-fitting-part base portion, and a recessed fitting part that is recessed from the outer-side-fitting-part base portion, and that is fitted with the protruding fitting part of the supported-part-side fitting part, the supported-part-side fitting part and the outer-side fitting part are fixed to the support member by the protruding fitting part being fitted with the recessed fitting part via the support member 10, and the supported-part-side fitting part and the supported part are integrally molded.

With the bioelectrode according to an aspect of the present invention, the snap-button-type connector and the supported part are integrally molded with the support member.

With the bioelectrode according to an aspect of the present invention, the electrode member is molded from conductive rubber containing silicone rubber and metal particles.

With the bioelectrode according to an aspect of the present invention, the snap-button-type connector is stainless steel.

To achieve the object described above, a manufacturing method of a bioelectrode according to the present invention is a manufacturing method of a bioelectrode including a support member, an electrode member of conductive rubber, including a supported part that is a member to be supported by the support member and at least one electrode part that is a member protruding from the supported part, and a snap-button-type connector that is provided on the support member and that electrically connects the electrode member to outside, the manufacturing method being characterized by including: a casting step of stirring the conductive rubber, and casting the conductive rubber into a shape of the electrode member; and a molding step of cross-linking the electrode member in a state in which the support member provided with the snap-button-type connector is placed on the supported part of the electrode member that is cast in the casting step, and integrally molding the snap-button-type connector and the supported part.

### Effects of Invention

With the bioelectrode and the manufacturing method of the bioelectrode according to the present invention, an electrode portion and a lead wire (connection wire) portion can be stably connected.

### Brief Description of Drawings

[Fig. 1] A perspective view schematically showing a structure of a bioelectrode according to an embodiment of the present invention.
[Fig. 2] A side view schematically showing the structure of the bioelectrode according to the embodiment of the present invention.
[Fig. 3] A plan view schematically showing the structure of the bioelectrode according to the embodiment of the present invention.
[Fig. 4] An A-A cross-sectional view schematically showing the structure of the bioelectrode shown in Fig. 2.
[Fig. 5] A view for describing a casting step and a molding step in a manufacturing method of the bioelectrode according to the embodiment of the present invention.
[Fig. 6] A view for describing a volume resistivity evaluation test for the bioelectrode according to the embodiment of the present invention.

### Description of Embodiments

Hereinafter, an embodiment of the present invention will be described with reference to the drawings.

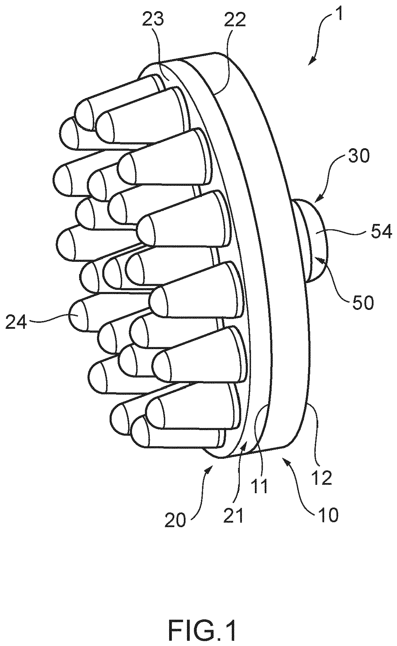

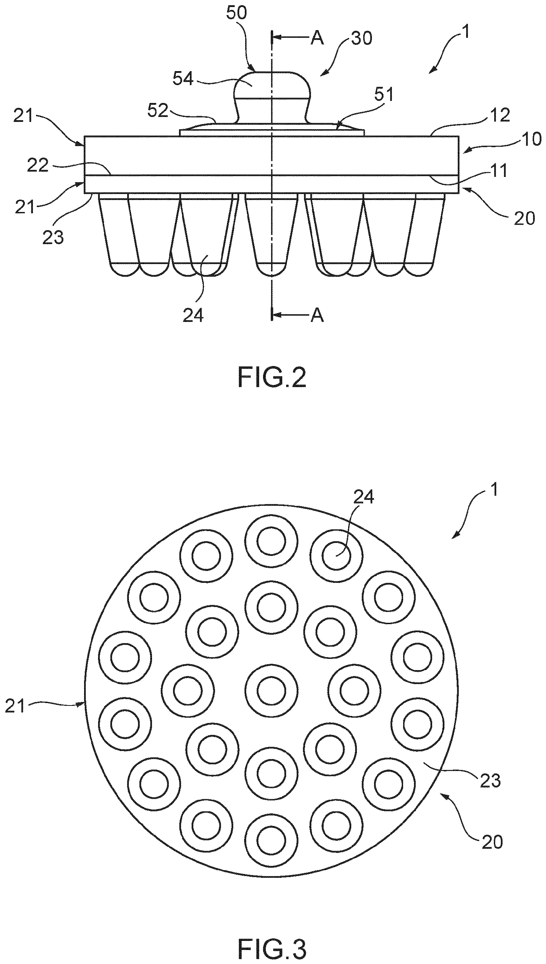

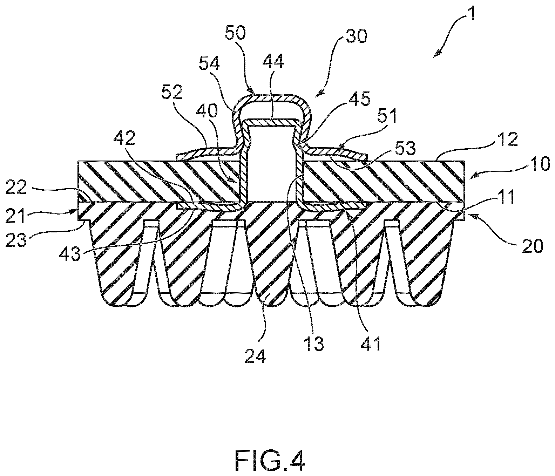

Fig. 1 is a perspective view schematically showing a structure of a bioelectrode 1 according to an embodiment of the present invention. Fig. 2 is a side view schematically showing the structure of the bioelectrode 1, and Fig. 3 is a plan view schematically showing the structure of the bioelectrode 1. Fig. 4 is an A-A cross-sectional view schematically showing the structure of the bioelectrode 1 shown in Fig. 2. The bioelectrode 1 according to the embodiment of the present invention includes a support member 10, an electrode member 20 of conductive rubber, including a supported part 21 that is a member supported by the support member 10 and at least one electrode part 24 that is a member protruding from the supported part 21, and a snap-button-type connector 30 that is provided on the support member 10, and that electrically connects the electrode member 20 to outside. The snap-button-type connector 30 and the supported part 21 are integrally molded.

The snap-button-type connector 30 includes a supported-part-side fitting part 40 provided on a side of the supported part 21, and a connection-surface-side fitting part 50 provided on a connection surface 12 that is an outer side. The supported-part-side fitting part 40 and the supported part 21 are integrally molded.

With the bioelectrode 1, a tip end portion of the electrode part 24 of the electrode member 20 contacts a body of a subject, and biosignals from the subject may be detected via the electrode part 24 of the electrode member 20. For example, the bioelectrode 1 is a bioelectrode for brain wave detection that detects brain waves by contacting a head of the subject. The bioelectrode 1 is not limited to brain wave detection, and may also be applied to other devices for detecting biosignals, such as a wearable information appliance. Hereinafter, a structure of the bioelectrode 1 will be specifically described.

As shown in Figs. 1 to 4, with respect to the bioelectrode 1, the support member 10 has a disk shape or a substantially disk shape, for example. The support member 10 includes a support surface 11 that is a surface for supporting the electrode member 20, and a connection surface 12 facing away from the support surface 11, the connection surface 12 being a surface that is on a side where a connection part (not shown) for enabling electrical connection to a measurement device (not shown) is to be connected. Additionally, the measurement device, not shown, is a device for receiving a biosignal detected by the bioelectrode 1, and for processing, analyzing, or displaying the biosignal that is received, for example. A through hole 13 (Fig. 4) penetrating the support surface 11 and the connection surface 12 is formed at a center or substantially at a center of the support member 10. The support member 10 supports the electrode member 20 with the support surface 11.

The support member 10 is formed of an insulating material, and is not electrically connected to the electrode member 20. For example, the support member 10 is formed of silicone rubber. Additionally, a shape of the support member 10 may be any shape and is not limited to a specific shape so long as the electrode member 20 can be supported.

The supported-part-side fitting part 40 (Fig. 4) of the snap-button-type connector 30 is provided at a center or substantially at a center of the support surface 11 of the support member 10. Details of the supported-part-side fitting part 40 of the snap-button-type connector 30 will be given later.

Furthermore, the connection-surface-side fitting part 50 of the snap-button-type connector 30 is provided at a center or substantially at a center of the connection surface 12 of the support member 10. Details of the connection-surface-side fitting part 50 of the snap-button-type connector 30 will be given later.

With the bioelectrode 1, the electrode member 20 includes the supported part 21 that is a member to be supported by the support member 10, and at least one electrode part 24 that is a member protruding from the supported part 21. The supported part 21 of the electrode member 20 has a disk shape or a substantially disk shape, for example. The supported part 21 includes a supported surface 22 that is a surface to be supported by the support surface 11 of the support member 10, and a protrusion surface 23 facing away from the supported surface 22, the protrusion surface 23 being a surface from which the electrode part 24 protrudes. When seen along an axial direction, the shape of the supported part 21 is a same or substantially same shape as the shape of the support member 10.

The bioelectrode 1 is provided with a plurality of electrode parts 24 on the electrode member 20, and the electrode parts 24 protrude in a same or substantially same directions from the protrusion surface 23 of the supported part 21. A tip end of each of the electrode parts 24 of the electrode member 20 is formed into a hemispherical or substantially hemispherical shape, for example. The electrode parts 24 of the electrode member 20 protrude from the protrusion surface 23 of the supported part 21 in a form of a brush, for example.

A shape of the electrode part 24 of the electrode member 20 is, as a whole, a conical or substantially conical shape that is tapered toward the tip end of the electrode part 24, for example. Additionally, the shape of the electrode part 24 of the electrode member 20 may be a shape that is columnar or substantially columnar and that includes a part that is tapered toward the tip end, and is not limited to a specific shape.

The electrode member 20 is formed of conductive rubber. The conductive rubber for forming the electrode member 20 includes silicone rubber and metal particles. For example, the silicone rubber is liquid silicone rubber of a room-temperature curing type, and the metal particles are silver particles. The metal particles may be of a carbonaceous material such as carbon black or carbon nanotube, for example, so long as the metal particles are of a metal material having conductivity.

The liquid silicone rubber of a room-temperature curing type is silicone rubber that is in a state of liquid or paste before curing, and that, normally, becomes a rubber elastic body when a curing reaction proceeds at 20°C to 100°C. The curing reaction may be a curing reaction that gradually proceeds due to moisture (water) in the air, or may be a curing reaction that is caused to swiftly proceed, by mixing a curing agent in a main material, and curing may be performed by any curing reaction. Furthermore, it is possible to use only one type of liquid silicone rubber of a room-temperature curing type, or it is possible to mix and use a plurality of types of liquid silicone rubber of a room-temperature curing type.

As the silver particles in the conductive rubber, those containing silver powder in an agglomerated form and silver powder in a flake form may be used. Silver powder in an agglomerated form is a plurality of primary particles in a particle form agglomerated into a three-dimensional form, and silver powder in a flake form has a shape that is scale-like. An average particle diameter of silver powder in an agglomerated form and silver powder in a flake form is not limited to a specific value.

Additionally, the conductive rubber for forming the electrode member 20 may further contain other components, in addition to components described above, so long as an advantageous effect of the present invention is not impaired. For example, as other components, additives commonly used in rubber industry, such as a reinforcing agent, a filler such as dry silica, an anti-aging agent, a processing aid, or a plasticizer, may be added as appropriate.

As described above, the electrode member 20 is formed by curing silicone rubber, and has flexibility and elasticity, good adhesion to a body of a subject, is soft on skin and does not cause discomfort even when adhered for a long time, and may maintain stable contact with the body of the subject.

With the bioelectrode 1, the snap-button-type connector 30 includes the supported-part-side fitting part 40 provided on the side of the supported part 21, and the connection-surface-side fitting part 50 provided on the connection surface 12, and the supported-part-side fitting part 40 and the supported part 21 are integrally molded. For example, the snap-button-type connector 30 is formed of stainless steel. Additionally, the snap-button-type connector 30 is not limited to be of a specific material so long as the supported-part-side fitting part 40 and the connection-surface-side fitting part 50 are electrically connected.

As shown in Fig. 4, with respect to the snap-button-type connector 30, the supported-part-side fitting part 40 includes a supported-part-side-fitting-part base portion 41, and a protruding fitting part 44 that is a member protruding from the supported-part-side-fitting-part base portion 41. With respect to the supported-part-side fitting part 40, the supported-part-side-fitting-part base portion 41 has, for example, a disk shape or a substantially disk shape, and is formed into a dish shape or a substantially dish shape that is shallowly recessed at a center or substantially at a center. The supported-part-side-fitting-part base portion 41 includes a protrusion surface 42 that is a surface from which the protruding fitting part 44 protrudes, and an embedded surface 43 that faces away from the protrusion surface 42 and that is to be embedded in the supported part 21 of the electrode member 20.

With respect to the supported-part-side fitting part 40, the protruding fitting part 44 protrudes from a center or substantially a center of the protrusion surface 42 of the supported-part-side-fitting-part base portion 41. The protruding fitting part 44 has a shape that is bottomed cylindrical or substantially bottomed cylindrical, and a fitting part 45 that is annular or substantially annular and that is recessed in a radial direction from an outer peripheral surface of the protruding fitting part 44 is formed at a tip end of the protruding fitting part 44. A diameter of the outer peripheral surface of the protruding fitting part 44 is same or substantially same as a diameter of an inner peripheral surface of the through hole 13 of the support member 10, and the protruding fitting part 44 is inserted in the through hole 13 of the support member 10. The protrusion surface 42 of the supported-part-side fitting part 40 is disposed on the support surface 11 of the support member 10, at the center or substantially at the center of the support surface 11 of the support member 10, with the protruding fitting part 44 inserted in the through hole 13 of the support member 10.

The supported-part-side fitting part 40 is integrally molded with the supported part 21 of the electrode member 20. Specifically, the supported-part-side fitting part 40 is provided at a center or substantially a center of the supported part 21 of the electrode member 20, with the embedded surface 43 of the supported-part-side-fitting-part base portion 41 embedded inside the supported part 21, on a side of the supported surface 22, and the supported-part-side-fitting-part base portion 41 of the supported-part-side fitting part 40 and the supported part 21 of the electrode member 20 are integrally molded. Additionally, only the embedded surface 43 of the supported-part-side-fitting-part base portion 41 is embedded inside the supported part 21, on the side of the supported surface 22, and the protrusion surface 42 of the supported-part-side-fitting-part base portion 41 is not embedded inside the supported part 21, on the side of the supported surface 22.

As shown in Fig. 4, with respect to the snap-button-type connector 30, the connection-surface-side fitting part 50 includes a connection-surface-side-fitting-part base portion 51, and a recessed fitting part 54 that is recessed from the connection-surface-side-fitting-part base portion 51, and that is formed to be capable of being fitted with the protruding fitting part 44 of the supported-part-side fitting part 40. With respect to the connection-surface-side fitting part 50, the connection-surface-side-fitting-part base portion 51 has a disk shape or a substantially disk shape, and is formed into a dish shape or a substantially dish shape that is shallowly recessed at a center or substantially at a center, for example.

The connection-surface-side-fitting-part base portion 51 includes a recessed surface 52 that is a surface from which the recessed fitting part 54 is recessed, and a facing surface 53 that faces away from the recessed surface 52 and that faces the connection surface 12 of the support member 10. A diameter of the connection-surface-side-fitting-part base portion 51 is same or substantially same as a diameter of the supported-part-side-fitting-part base portion 41.

With respect to the connection-surface-side fitting part 50, the recessed fitting part 54 is recessed from a center or substantially a center of the recessed surface 52 of the connection-surface-side-fitting-part base portion 51. The recessed fitting part 54 has a shape that is bottomed cylindrical or substantially bottomed cylindrical, is gradually widened in a radial direction from a base of the recessed fitting part 54 toward a tip end, and is fitted with the fitting part 45 of the protruding fitting part 44 of the supported-part-side fitting part 40 at the base of the recessed fitting part 54.

That is, a diameter of the base of the recessed fitting part 54 is same or substantially same as a diameter of the fitting part 45 of the protruding fitting part 44 of the supported-part-side fitting part 40, and the recessed fitting part 54 is fitted, at the center or substantially the center of the connection surface 12 of the support member 10, with the fitting part 45, of the protruding fitting part 44 of the supported-part-side fitting part 40, that is inserted in the through hole 13 of the support member 10. In this manner, the supported-part-side fitting part 40 and the connection-surface-side fitting part 50 are fixed on the support member 10, by the protruding fitting part 44 being fitted with the recessed fitting part 54 via the support member 10 by crimping or the like, for example. That is, the supported-part-side fitting part 40 and the connection-surface-side fitting part 50 are fixed on the support member 10, by the protruding fitting part 44 and the recessed fitting part 54 being fitted together in a state where the support member 10 is sandwiched between the supported-part-side-fitting-part base portion 41 and the connection-surface-side-fitting-part base portion 51.

The connection-surface-side fitting part 50 functions as a terminal for electrically connecting the bioelectrode 1 to the measurement device (not shown) described above. For example, the connection-surface-side fitting part 50 is connected by being fitted with a connection part (not shown), of another snap-button-type connector or the like corresponding to the snap-button-type connector 30, that allows electrical connection to the measurement device (not shown) described above. Additionally, it is enough if the connection-surface-side fitting part 50 can be electrically connected to outside, to a lead wire, an external appliance, or a measurement appliance, for example, without being limited to the measurement device (not shown).

Next, a manufacturing method of the bioelectrode 1 having the structure described above will be described. The manufacturing method of the bioelectrode 1 includes a casting step and a molding step. The casting step is a step of stirring conductive rubber and casting the conductive rubber into a shape of the electrode member 20, and the molding step is a step of cross-linking the electrode member 20 by placing the support member 10 provided with the snap-button-type connector 30 on the supported part 21 of the electrode member 20 that is cast in the casting step, and integrally molding the snap-button-type connector 30 and the supported part 21. Hereinafter, the manufacturing method of the bioelectrode 1 will be specifically described.

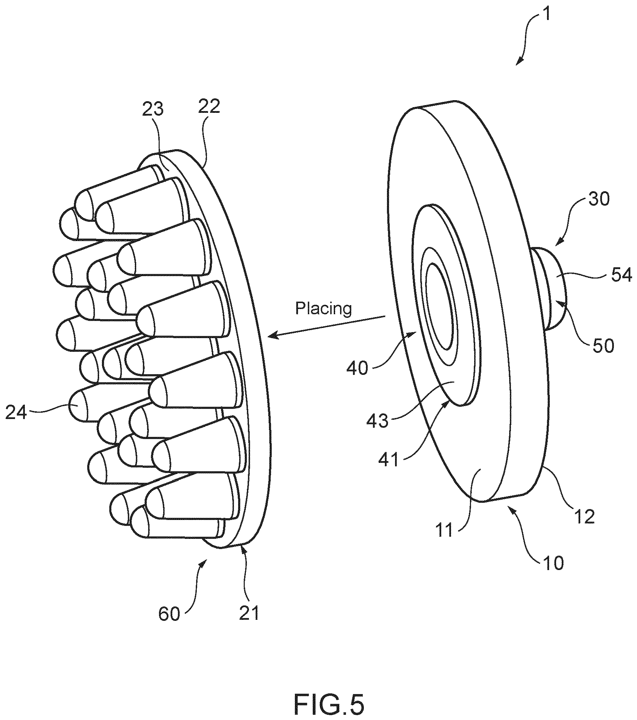

Fig. 5 is a view for describing the casting step and the molding step in the manufacturing method of the bioelectrode 1 according to the embodiment of the present invention. As shown in Fig. 5, in the casting step, conductive rubber containing silicone rubber and metal particles is stirred, and the conductive rubber is injected in a mold (cavity) having the shape of the electrode member 20, and an intermediate product 60 where the supported part 21 and the electrode part 24 of the electrode member 20 are molded is cast. With the intermediate product 60, the embedded surface 43 of the supported-part-side fitting part 40 of the snap-button-type connector 30 is not embedded in the supported part 21 of the electrode member 20.

Next, as shown in Fig. 5, in the molding step, the support member 10 in a state where the supported-part-side fitting part 40 of the snap-button-type connector 30 is fitted, on the support surface 11 of the support member 10, with the connection-surface-side fitting part 50 and fixed to the support member 10 is placed on the supported surface 22 of the supported part 21 of the intermediate product 60 that is cast in the mold (cavity).

Next, in the molding step, the intermediate product 60 is cross-linked in a state where the support member 10 is placed on the supported surface 22 of the supported part 21 of the intermediate product 60. The curing is thereby performed in a state in which the embedded surface 43 of the supported-part-side-fitting-part base portion 41 of the supported-part-side fitting part 40 is embedded inside the supported part 21, on the side of the supported surface 22, and the supported-part-side fitting part 40 and the supported part 21 are integrally molded. Additionally, the protrusion surface 42 of the supported-part-side-fitting-part base portion 41 is not embedded inside the supported part 21, on the side of the supported surface 22.

The electrode member 20 is molded by curing silicone rubber as a binder in which metal particles are added, and a molding surface layer (not shown) that is a layer with a small amount of metal particles is formed on a surface of the electrode member 20 that is molded. Contact impedance between the embedded surface 43 of the supported-part-side-fitting-part base portion 41 and the supported part 21 of the electrode member 20 is specified not by an apparent contact area, but by an effective contact area, for achieving electrical contact, between the embedded surface 43 of the supported-part-side-fitting-part base portion 41 and metal particles in the supported part 21.

In a case where a surface of the embedded surface 43 of the supported-part-side-fitting-part base portion 41 and a surface of the supported surface 22 of the supported part 21 are in contact, or in other words, in a case where the surfaces are attached without the embedded surface 43 of the supported-part-side-fitting-part base portion 41 being embedded in the supported surface 22 of the supported part 21, the embedded surface 43 of the supported-part-side-fitting-part base portion 41 contacts the molding surface layer (not shown), and the contact impedance is increased, and noise that is mixed in a biosignal that is detected is possibly increased, or acquisition of the biosignal is possibly made impossible.

In contrast, the embedded surface 43 of the supported-part-side-fitting-part base portion 41 of the supported-part-side fitting part 40 is embedded inside the supported part 21, on the side of the supported surface 22, and the embedded surface 43 contacts the supported part 21 on an inner side than the molding surface layer (not shown) without contacting the molding surface layer (not shown) on the surface of the supported surface 22 of the supported part 21. Accordingly, the contact impedance between the electrode member 20 and the supported-part-side fitting part 40 may be reduced, and noise that is mixed in a biosignal that is detected is not increased, and also, acquisition of the biosignal is not made impossible.

Furthermore, according to the manufacturing method of the bioelectrode 1, the curing is performed in a state in which the embedded surface 43 of the supported-part-side-fitting-part base portion 41 of the supported-part-side fitting part 40 is embedded inside the supported part 21, on the side of the supported surface 22. Accordingly, the supported part 21 of the electrode member 20 is formed in accordance with asperities of the embedded surface 43 of the supported-part-side-fitting-part base portion 41 of the supported-part-side fitting part 40, and the supported part 21 of the electrode member 20 makes a surface contact with the asperities of the embedded surface 43 of the supported-part-side-fitting-part base portion 41.

The effective contact area that is a contact area between the embedded surface 43 of the supported-part-side-fitting-part base portion 41 and the metal particles in the supported part 21 may thus be increased, and as a result, the contact impedance between the electrode member 20 and the supported-part-side fitting part 40 may be reduced. Furthermore, because the supported-part-side fitting part 40 and the supported part 21 are integrally molded, an interface between the embedded surface 43 of the supported-part-side-fitting-part base portion 41 and the metal particles in the supported part 21 does not move, and stable contact impedance may be achieved.

As described above, with the bioelectrode 1 according to the embodiment of the present invention, the snap-button-type connector 30 includes the supported-part-side fitting part 40 provided on the side of the supported part 21, and the connection-surface-side fitting part 50 provided on the connection surface 12. Due to the protruding fitting part 44 of the supported-part-side fitting part 40 being inserted in the through hole 13 of the support member 10 and the fitting part 45 being fitted with the base of the recessed fitting part 54 of the connection-surface-side fitting part 50, the support member 10 and the electrode member 20 are fixed, and the supported-part-side fitting part 40 and the supported part 21 are integrally molded.

Accordingly, the bioelectrode 1 may be electrically connected to the measurement device (not shown) described above via the connection-surface-side fitting part 50, due to the connection-surface-side fitting part 50 being fitted with a connection part (not shown) of another snap-button-type connector or the like, of the measurement device (not shown) described above, corresponding to the snap-button-type connector 30. A connection portion between an electrode portion and a connection wire portion may thus be prevented from becoming unstable, and the connection portion between the electrode portion and the connection wire portion may be prevented from coming apart due to movement of the subject or the like, and the electrode portion and the connection wire portion may be stably connected.

Furthermore, with the bioelectrode 1, the electrode part 24 of the electrode member 20 is formed of conductive rubber, and has desirable elasticity such that no discomfort is felt by the subject, and also, the electrode part 24 may be evenly adhered to a target part of the subject. Accordingly, a reinforcing member such as a core material for giving elasticity to the electrode part 24 of the electrode member 20 is not necessary. Furthermore, the electrode part 24 of the electrode member 20 may be formed with only conductive rubber, and a structure of the electrode member 20 is not complex and manufacturing is facilitated. Moreover, with the bioelectrode 1, the snap-button-type connector 30 is formed of stainless steel, and thus has good conductivity and is easily handled, and the electrode portion and the connection wire portion may be stably connected at a low cost.

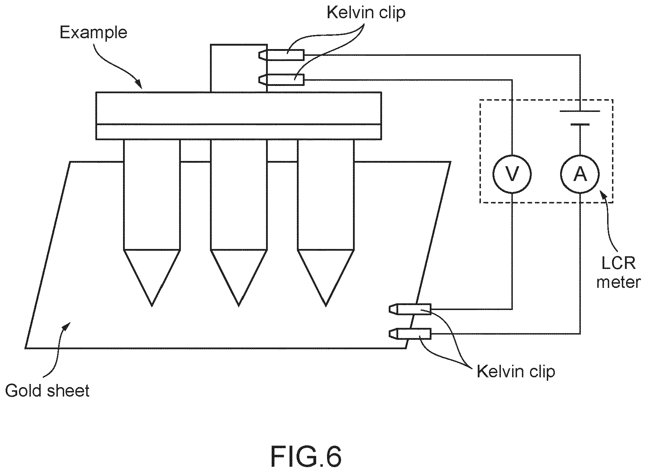

Next, an evaluation test for volume resistivity of the bioelectrode 1 according to the embodiment of the present invention will be described. The present inventor fabricated the bioelectrode 1 according to the embodiment of the present invention described above (example), and performed a volume resistivity evaluation test on the bioelectrode 1. As shown in Fig. 6, the volume resistivity evaluation test was performed by measuring volume resistivity of the example by using an LCR meter. Specifically, the bioelectrode 1 (example) was placed on a gold sheet and was connected with Kelvin clips, and the volume resistivity was measured by the LCR meter. Additionally, a following LCR meter was used as the LCR meter.

### - LCR meter: ZM2371 manufactured by NF Corporation

Furthermore, with respect to the example, conductive rubber of components indicated in Table 1 below was centrifugally stirred, and was injected into a mold (cavity). Then, the support member 10 in a state where the supported-part-side fitting part 40 of the snap-button-type connector 30 was fitted, on the support surface 11 of the support member 10, with the connection-surface-side fitting part 50 and fixed to the support member 10 was placed on the supported surface 22 of the supported part 21 of the intermediate product 60 that was cast in the mold (cavity). Next, the intermediate product 60 was cross-linked under cross-linking conditions indicated in Table 2, in a state where the support member 10 was placed on the supported surface 22 of the supported part 21 of the intermediate product 60. Then, brine treatment was performed in an autoclave under brine treatment conditions indicated in Table 3, and the bioelectrode 1 of the example was fabricated.

**[Table 1]**

| | Used product (used material) | Added amount |
|---|---|---|
| Liquid silicone rubber of room-temperature curing type | Manufactured by Shin-Etsu Chemical Co., Ltd. KE-106 | 100phr |
| Curing agent | Manufactured by Shin-Etsu Chemical Co., Ltd. CAT-RG | 10phr |
| Silver particle | Manufactured by DOWA HOLDINGS CO., LTD. G-35 | 165phr |
| | Manufactured by DOWA HOLDINGS CO., LTD. FA-2-3 | 165phr |
| Dispersing agent | Manufactured by Shin-Etsu Chemical Co., Ltd. KF-6106 | 10phr |
| | Manufactured by Shin-Etsu Chemical Co., Ltd. KF-6015 | 10phr |

**Table 2]**

| | Example |
|---|---|
| Primary cross-linking | 150°C, 3min |
| Secondary cross-linking | 150°C, 30min |

**Table 3]**

| | Example |
|---|---|
| Brine concentration | 10% |
| Immersion time | 1 hr |
| Brine temperature | 121°C |
| Pressure | 0.1 MPaG |

In the present evaluation test, the volume resistivity was measured in the manner described above for the example by using 10 samples. A test result is an average value of measured values for the 10 samples. The measurement result of the volume resistivity for the example was 0.98 Ω. In this manner, it was confirmed that the electrode member 20 is electrically connected via the snap-button-type connector 30.

Heretofore, an embodiment of the present invention has been described, but the present invention is not limited to the embodiment of the present invention described above, and includes any mode within the concept of the present invention and the scope of the claims. Furthermore, structures may be selectively combined as appropriate to achieve at least one of objects and advantageous effects described above. For example, the shape, material, arrangement, size and the like of each structural element in the embodiment described above may be changed as appropriate according to a specific usage mode of the present invention.

For example, the support member 10 is not limited to the shape described above, and may take other shapes. Likewise, the electrode member 20 is not limited to the shape described above, and may take other shapes.

Furthermore, the supported-part-side fitting part 40 and the connection-surface-side fitting part 50 may both be formed from a same material using the stainless steel mentioned above, or may be formed from different materials. In the case of forming the supported-part-side fitting part 40 and the connection-surface-side fitting part 50 from different materials, the supported-part-side fitting part 40 and the connection-surface-side fitting part 50 may be formed from materials having conductivity and different from stainless steel. As the materials having conductivity for the supported-part-side fitting part 40 and the connection-surface-side fitting part 50, different metals such as copper and aluminum may be used, for example. The materials for the supported-part-side fitting part 40 and the connection-surface-side fitting part 50 are not limited to the above, and materials having conductivity, such as conductive rubber, may be used.

Furthermore, a case has been described where the supported-part-side fitting part 40 and the connection-surface-side fitting part 50 are fixed to the support member 10 by being fitted together by crimping or the like, for example. However, the present invention is not limited to such a case, and the supported-part-side fitting part 40 may be provided on the electrode member 20 and the connection-surface-side fitting part 50 may be provided on the support member 10, and the supported-part-side fitting part 40 and the connection-surface-side fitting part 50 may be fixed to the support member 10 by being fitted together by crimping or the like, for example.

Moreover, a case has been described where only the embedded surface 43 of the supported-part-side-fitting-part base portion 41 is provided embedded in the supported surface 22 of the supported part 21. However, the present invention is not limited to such a case, and all of the supported-part-side-fitting-part base portion 41 may be embedded in the supported surface 22 of the supported part 21. Furthermore, the connection-surface-side fitting part 50 may be provided, at the center or substantially the center of the connection surface 12 of the support member 10, with an outer edge of the facing surface 53 of the connection-surface-side fitting part 50 adhered to the connection surface 12 of the support member 10 with an adhesive or the like, or may be provided partly being embedded inside the connection surface 12 of the support member 10.

### List of Reference Signs

- 1: bioelectrode,
- 10: support member,
- 11: support surface,
- 12: connection surface,
- 13: through hole,
- 20: electrode member,
- 21: supported part,
- 22: supported surface,
- 23: protrusion surface,
- 24: electrode part,
- 30: snap-button-type connector,
- 40: supported-part-side fitting part,
- 41: supported-part-side-fitting-part base portion,
- 42: protrusion surface,
- 43: embedded surface,
- 44: protruding fitting part,
- 45: fitting part,
- 50: connection-surface-side fitting part,
- 51: connection-surface-side-fitting-part base portion,
- 52: recessed surface,
- 53: facing surface,
- 54: recessed fitting part

## Claims

1. A bioelectrode **characterized by** comprising:
a support member;
an electrode member of conductive rubber, including a supported part that is a member to be supported by the support member, and at least one electrode part that is a member protruding from the supported part; and
a snap-button-type connector that is provided on the support member, and that electrically connects the electrode member to outside, wherein
the snap-button-type connector and the supported part are integrally molded.

2. The bioelectrode according to claim 1, **characterized in that**:
the snap-button-type connector includes a supported-part-side fitting part provided on a side of the supported part, and an outer-side fitting part provided on an outer side,
the supported-part-side fitting part includes a supported-part-side-fitting-part base portion, and a protruding fitting part that is a member protruding from the supported-part-side-fitting-part base portion,
the outer-side fitting part includes an outer-side-fitting-part base portion, and a recessed fitting part that is recessed from the outer-side-fitting-part base portion, and that is fitted with the protruding fitting part of the supported-part-side fitting part,
the supported-part-side fitting part and the outer-side fitting part are fixed to the support member by the protruding fitting part being fitted with the recessed fitting part via the support member, and
the supported-part-side fitting part and the supported part are integrally molded.

3. The bioelectrode according to claim 1 or 2, **characterized in that** the snap-button-type connector and the supported part are integrally molded with the support member.

4. The bioelectrode according to any one of claims 1 to 3, **characterized in that** the electrode member is molded from conductive rubber containing silicone rubber and metal particles.

5. The bioelectrode according to any one of claims 1 to 4, **characterized in that** the snap-button-type connector is stainless steel.

6. A manufacturing method of a bioelectrode including a support member, an electrode member of conductive rubber, including a supported part that is a member to be supported by the support member and at least one electrode part that is a member protruding from the supported part, and a snap-button-type connector that is provided on the support member and that electrically connects the electrode member to outside, the manufacturing method being **characterized by** comprising:
a casting step of casting the conductive rubber into a shape of the electrode member; and
a molding step of cross-linking the electrode member in a state in which the support member provided with the snap-button-type connector is placed on the supported part of the electrode member that is cast in the casting step, and integrally molding the snap-button-type connector and the supported part.
